(19) Europäisches Patentamt
European Patent Office
Office européen des brevets

(11) Publication number: 0 142 868 B1

# (12) EUROPEAN PATENT SPECIFICATION

(45) Date of publication of patent specification: 01.06.88

(51) Int. Cl.⁴: **C 07 C 85/08**, C 07 C 87/04, C 07 C 87/14

(21) Application number: 84114133.6

(22) Date of filing: 22.11.84

## (54) Process for producing tertiary amines.

(30) Priority: 22.11.83 JP 218689/83
19.09.84 JP 194758/84

(43) Date of publication of application:
29.05.85 Bulletin 85/22

(45) Publication of the grant of the patent:
01.06.88 Bulletin 88/22

(84) Designated Contracting States:
BE DE FR GB IT NL

(56) References cited:
BE-A- 672 711
FR-A-2 231 663
FR-A-2 289 486
FR-A-2 349 565
GB-A-2 025 408
US-A-4 207 260

(73) Proprietor: NIPPON OIL AND FATS COMPANY, LIMITED
10-1, Yuraku-cho 1-chome
Chiyoda-ku Tokyo (JP)

(72) Inventor: Koyama, Motoo
No. 7-284, Otodacho
Ikoma-shi Nara (JP)
Inventor: Takahashi, Fujio
No. 4-12-1, Oshonishimachi
Amagasaki-shi Hyogo (JP)
Inventor: Noji, Yukihiro
No. 4-12-1, Oshonishimachi
Amagasaki-shi Hyogo (JP)
Inventor: Niiyama, Kaoru
No. 2-53-1-304, Mukonosonishi
Amagasaki-shi Hyogo (JP)
Inventor: Miyata, Toyomi
No. 2-53-1-402, Mukonosonishi
Amagasaki-shi Hyogo (JP)

(74) Representative: Hansen, Bernd, Dr.rer.nat. et al
Hoffmann, Eitle & Partner Patentanwälte
Arabellastrasse 4
D-8000 München 81 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 142 868 B1

Courier Press, Leamington Spa, England.

**Description**

The present invention relates to a process for producing tertiary amines. More particularly, the invention relates to a process for producing tertiary amines of high purity and quality in high yield by reductively alkylating primary or secondary amines with hydrogen and an aldehyde in the presence of certain hydrogenating catalysts.

Aliphatic tertiary amines are useful as corrosion inhibitors and oil additives. They are also useful as intermediates for the production of quaternary ammonium salts and amphoteric surface active agents such as disinfectants, fungicides, sanitary agents, retarders in dyeing, and antistatic agents. As the use of aliphatic tertiary amines is expanding today, the requirements for their quality as intermediates are becoming increasingly stringent. Among other things, tertiary amines are required to have minimum levels of impurities that may impart undesired colors or smells to the final product.

Various methods are known for methylating primary or secondary amines: (A) methylation with formic acid and formaldehyde (*Organic Syntheses,* Collective Volume 4, p. 723, John Wiley & Sons, Inc. (1963)), (B) methylation with hydrogen and formaldehyde (*Organic Syntheses,* Vol. 4, p. 174, John Wiley & Sons, Inc. (1963)). Higher alkyl methylamines may be produced by (C) reaction between higher alkyl halides and methylamine (U.S. Patent 3,379,764) or (D) reductive amination between higher alcohols and methylamine (Japanese Patent Application (OPI) No. 19604/1977, Japanese Patent Publication Nos. 849/1982 and 55704/1982) (the symbol "OPI" as used herein means an unexamined published Japanese patent application).

Conventionally, the higher alkyl dimethylamines are manufactured by method (C), which involves a reaction that entirely differs from the one used in the process of the present invention. In method (D), higher aldehyde produced by the dehydrogenation of a higher alcohol is reacted with methylamine. However, because of the high reaction temperatures involved and the high yield of by-products, the quality of the tertiary amines produced by method (D) is very poor.

Method (A) is unable to give a sufficient conversion of formaldehyde unless formic acid is used in large excess. Since formic acid is expensive, this method is not commercially useful except in the case of producing special tertiary amines.

Method (B) uses the same type of reaction as is employed in the process of the present invention. This method has so far been considered unsuitable for implementation on an industrial scale because the reaction yield and the quality of the product are much inferior to those obtained in method (C). However, researchers have made various efforts to improve this method since it has potentially a wide scope of applications, and an increase in the reaction yield and the product quality might render the method cost-effective. An example of the studies made along this line is shown in *Organic Reactions*, Vol. 4, p. 244 and p. 248, John Wiley & Sons, Inc. (1948). According to this paper, yields no higher than 90% were obtained when primary or secondary amines were reacted with hydrogen and formaldehyde in the presence of Raney nickel or platinum catalyst. United States Patent No. 3,136,819 shows a method for improving a yield by using an additional catalyst in the reaction such as a short chain monocarboxylic acid or a short chain hydroxy monocarboxylic acid. However, when a primary amine having an alkyl group of 8 or more carbon atoms was subjected to reaction in the presence of a Raney nickel catalyst, the reaction yield was low and the resulting product contained only about 85% of a tertiary amine.

The present inventors have made detailed studies with respect to the alkylation of primary or secondary amines with hydrogen and an aldehyde, and obtained the following observations.

When the primary or secondary amine is reacted with an aldehyde, 1-hydroxyalkylamine

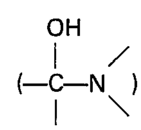

is formed as an intermediate, and an imine (—CH=N—) is also formed as a result of intramolecular dehydration. These are highly reactive compounds. However, if the reaction system has an insufficient reductive action, the hydrogenation of 1-hydroxyalkylamine or imine is unlikely to occur; instead, they react with the primary or secondary amine present in a large amount in the reaction system, thereby forming an unstable polymer such as polyalkylene-polyamine which is deposited on the hydrogenation catalyst and inhibits the diffusion of the amine into the catalyst surface. In an extreme case, 1-hydroxyalkylamine or imine may also react with the active methylene group of the resulting polyalkylenepolyamine. The aldehyde may also react with this active methylene group. According to the finding of the present inventors, the aldehyde reacted with 1-hydroxyalkylamine as well as the polyalkylenepolyamine, and the resulting complex by-products not only reduced the yield of the intended tertiary amine, but also caused a malodor, stain, and gradual coloration in the amine.

Based on these observations, the present inventors made various studies with a view toward obtaining a hydrogenation catalyst that satisfied the following three requirements; good dispersion in the amine layer (reactive phase), high stability of the dispersion against moisture, and great hydrogenating ability. The present invention has been accomplished as a result of these studies.

The present invention provides a process for producing a tertiary amine by alkylating an amine of formula (I)

$$R^1R^2N[(CH_2)_nNH]_mR^3 \quad (I)$$

wherein $R^1$ represents a linear or branched alkyl or alkenyl group having from 8 to 24 carbon atoms; $R^2$ and $R^3$ each represents a hydrogen atom, or a linear or branched alkyl or alkenyl group having from 8 to 24 carbon atoms; m is 0 or an integer of 1 to 5 and n is 2 or 3, provided that when m=0, at least one of $R^2$ and $R^3$ represents a hydrogen atom with an aldehyde of formula (II)

$$R^4\overset{\overset{\displaystyle O}{\|}}{C}H \quad (II)$$

wherein $R^4$ represents a hydrogen atom, a saturated or unsaturated, linear, branched or cyclic aliphatic hydrocarbon group having from 1 to 24 total carbon atoms, an aromatic group-substituted aliphatic hydrocarbon group having 24 or less total carbon atoms, or a substituted or unsubstituted aromatic hydrocarbon group having 24 or less total carbon atoms.

According to the present invention, a hydrogenation catalyst having from 0.1 to 10 wt% of at least one of Co, Ni, Ru, Rh, Pd, and Pt supported on pulverized or granular carbon is provided in a reaction zone at the concentration of from 5 to 5,000 ppm of the catalyst metal based on the amount of the amine of formula (I), and the reaction is performed at a temperature of from 80 to 250°C and a hydrogen pressure of at least 2 kg/cm$^2$ (gauge) while the aldehyde is continuously supplied to the reaction zone.

Illustrative amines of formula (I) include octylamine, dodecylamine, tetradecylamine, hexadecylamine, octacdecylamine, docosylamine, oleylamine, linolylamine, and erucylamine. Amine mixtures may also be used, including cocoalkyl amine, tallowalkyl amine, hydrogenated tallowalkyl amine, rapeseed-oil-alkylamine, dicocoalkyl amine, ditallowalkyl amine, dihydrogenated tallowalkyl amine, 2-aminoethyl cocoalkyl amine, 2-aminoethyl tallowalkyl amine, 3-aminopropyl cocoalkyl amine, 3-aminopropyl tallowalkyl amine, N,N'-dicocoalkyl ethylenediamine, N-cocoalkyl N'-tallowalkyl ethylenediamine, N-cocoalkyl diethylenetriamine, N-tallowalkyl diethylenetriamine, N-cocoalkyl dipropylenetriamine, N-tallowalkyl dipropylenetriamine, N-cocoalkyl tripropylenetetramine, N-tallowalkyl tripropylenetetramine, N-cocoalkyl tetrapropylenepentamine, N-tallowalkyl tetrapropylenepentamine, N-cocoalkyl pentapropylenehexamine, and N-tallowalkyl pentapropylenehexamine. These amines may be used either individually or in combination.

Illustrative aldehydes of formula (II) include saturated or unsaturated, linear, branched or cyclic aliphatic aldehydes such as formaldehyde, acetaldehyde, propanal, hexanal, 2-ethylhexanal, 2-hexenal, 2-nonenal and cyclohexylaldehyde; aromatic group-substituted aliphatic aldehydes such as phenylacetaldehyde and 3-phenylpropanal; aromatic aldehydes such as benzaldehyde, o-, m- or p-tolualdehyde, p-methoxybenzaldehyde, p-chlorobenzaldehyde and 1-naphthylaldehyde. These aldehydes may be used either independently or in combination. The aldehydes may be used as an aqueous solution or a non-reactive solvent solution [examples of the solvent including an alcohol (e.g., methanol, ethanol), an ether (e.g., tetrahydrofuran) and a hydrocarbon (e.g., hexane, cyclohexane)], if desired.

The aldehyde is used in an amount of from 1 to 1.5 mols, and preferably 1 to 1.05 mols, per mol of active hydrogen on the amino or imino group of a primary or secondary amine. If the amount of the aldehyde used is less than 1 mol per mol of the active hydrogen defined above, the primary or secondary amine remains unreacted in the final product. If the amount of the aldehyde is more than 1.5 mols per mol of the active hydrogen, the process is not cost-effective and an extra and prolonged step is required for removing the residual aldehyde by reduction.

The hydrogenation catalyst used according to the present invention comprises from 0.1 to 10 wt% of Ni, Co, Ru, Rh, Pd or Pt as supported on pulverized or granular carbon. Such a hydrogenation catalyst may be prepared by any conventional method, such as the one shown in *Advances in Catalysis,* Vol. 20, p. 112 (1969). The hydrogenation catalyst is used in the present invention at the concentration of 5 to 5,000 ppm of the catalyst metal based on the amount of the starting amine. Even in a reaction system comprising both water and amine layers, the hydrogenation catalyst shown above is dispersed predominantly into the amine layer, and its dispersion into the aqueous layer is negligible.

Alumina, silica, and diatomaceous earth are not desired as supports for the hydrogenation catalyst since they do not provide a good dispersion of the catalyst in the reaction system concerned. Metals and metal oxides such as Raney nickel, Raney cobalt, platinum oxide, platinum black, and palladium black are known as hydrogenation catalysts having an extremely high activity, but they do not provide good results in the reaction of the present invention.

The process of the present invention uses an autoclave and performs the intended reaction at a hydrogen pressure of at least 2 kg/cm$^2$ (gauge), preferably from 5 to 50 kg/cm$^2$ (gauge), and at a temperature of from 80 to 250°C, and preferably from 100 to 250°C. If the hydrogen pressure is less than 2 kg/cm$^2$ (gauge) or if the reaction temperature is less than 80°C, the desired hydrogenation does not proceed satisfactorily, and instead, by-products such as 1-hydroxyalkylamine or its polymer and imine are formed

in increasing amounts. If the reaction temperature exceeds 250°C, by-products such as a polyalkylamine and hydrocarbon are formed in increasing amounts due to the dehydrogenation of the starting amine.

The aldehyde may be added by any method that permits its continuous or periodic addition. Usually, the aldehyde is supplied in small portions into the reaction zone of a reactor by means of a compressor.

If an aqueous solution of the aldehyde is likely to cause a great accumulation of water during the reaction, or if the reaction is expected to produce a large amount of water as a by-product, hydrogen may be released or circulated so as to discharge water out of the system while the reaction is being carried out.

An illustrative method for implementing the present invention is shown below. An autoclave equipped with a stirrer, a compressor, and an optional gas circulator with a cooling condenser is charged with the starting amine and a hydrogenation catalyst. The temperature in the autoclave is elevated to the desired level under stirring, and after the atmosphere of the reactor is replaced by hydrogen, an additional amount of hydrogen is supplied to a predetermined pressure. Subsequently, the aldehyde is fed into the autoclave under pressure, and the amine is reductively alkylated at the predetermined hydrogen pressure. When the removal of the accumulated water from the reaction system is desired to decrease the partial pressure of the water in the reaction system and to accelerate the reaction, a hydrogen gas circulator equipped with a cooling condenser is used to discharge the condensed water out of the system. After all of the aldehyde has been fed into the reactor, the reaction is continued for a given period while the temperature and hydrogen pressure are held constant.

If amine alkylation is performed by the method of the present invention, the intended reaction proceeds in a substantially quantitative manner and the formation of by-products that may impair the quality of the end compound is negligible. Therefore, a crude tertiary amine product that is substantially colorless and odorless is obtained. The amine alkylation ratio is at least 97% irrespective of the type of the starting primary or secondary amine. The distillation yield is 95% or more, and a tertiary amine having a purity of 98% or higher can be obtained.

The tertiary amine produced by the method of the present invention is highly stable against heat and light; it withstands a heat resistance test at 50°C and a daylight exposure test for at least 6 months without experiencing any change in color or smell.

This tertiary amine can be used to prepare amine oxides and quaternary ammonium salts. All the products are substantially colorless and odorless and have no problems at all in appearance or quality.

The advantages of the present invention will become more apparent by reading the following working examples and comparative examples.

Examples 1 to 17

An autoclave (capacity: 2 liters) equipped with a turbine stirrer and a compressor was charged with a starting primary or secondary amine (800 g in Examples 1 to 9, and 400 g in Examples 10 to 17) and a hydrogenation catalyst (for its type, see Table 1). The temperature in the autoclave was elevated to a predetermined level under agitation at 900 rpm with the turbine stirrer. The atmosphere of the autoclave was replaced by hydrogen, and an additional volume of hydrogen was supplied to a predetermined level.

While an aldehyde was pumped into the autoclave over a predetermined period, the starting amine was reductively alkylated at a predetermined temperature and hydrogen pressure. After the completion of the addition of the aldehyde, the reaction was continued for 30 minutes. After completion of the reaction, the hydrogenation catalyst was filtered off and the aqueous layer was separated to obtain a crude tertiary amine. In some Examples, the crude tertiary amine was purified by vacuum distillation. The reaction conditions used in the respective Examples and the analytical values for the resulting products are shown in Table 1.

As is clear from Table 1, all the crude tertiary amines obtained were substantially colorless (APHA<30). The purified tertiary amines were completely colorless and transparent. None of the purified and crude tertiary amines had a smell other than that of amines. The crude tertiary amines had a purity of at least 98%, whereas the purified tertiary amines were at least 99% pure. The purified tertiary amines were stored at 50°C for 6 months. They were also subjected to a daylight exposure test outdoors for 6 months. In either test, the amines experienced no change in appearance or smell.

TABLE 1-1

| | Example No. 1 | 2 | 3 | 4 | 5 | 6 | 7 | 8 | 9 |
|---|---|---|---|---|---|---|---|---|---|
| Starting amine | Dodecylamine | Dodecylamine | Dodecylamine | Octadecyl-amine | Tallow-alkylamine | Dicocoalkyl-amine | Dicocoalkyl-amine | Dicocoalkyl-amine | Dihydrogenated tallowalkylamine |
| Reaction condition | | | | | | | | | |
| Hydrogenation catalyst | | | | | | | | | |
| Type | 5% Pd-C | 5% Pd-C | 2% Pd-C | 5% Pd-C | 0.5% Pd-C | 10% Ni-C | 10% Co-C | 5% Pd-C | 10% Ni-C |
| Amount (%)[1] | 0.1 (50) | 0.4 (200) | 0.1 (20) | 0.05 (25) | 0.1 (5) | 5 (5,000) | 5 (5,000) | 0.01 (5) | 5 (5,000) |
| Aldehyde[2] | | | | | | | | | |
| Type | Formaldehyde | Formaldehyde | Formaldehyde | Formaldehyde | Formaldehyde | Formaldehyde | Formaldehyde | Formaldehyde | Formaldehyde |
| Amount | 1.05 | 1.0 | 1.05 | 1.0 | 1.0 | 1.0 | 1.0 | 1.0 | 1.0 |
| Reaction temperature (°C) | 145 | 160 | 145 | 145 | 145 | 180 | 200 | 250 | 180 |
| Reaction pressure ($kg/cm^2 \cdot G$) | 15 | 5 | 30 | 30 | 20 | 30 | 30 | 50 | 15 |
| Reaction time (hr) | 6 | 4 | 5 | 4 | 6 | 5 | 6 | 4 | 4 |
| Analytical value of product | | | | | | | | | |
| Tertiary amine | N,N-dimethyl-dodecylamine | N,N-Dimethyl-dodecylamine | N,N-Dimethyl-dodecylamine | N,N-Dimethyl-octadecyl-amine | N,N-Dimethyl-tallowalkyl-amine | N,N-Dicoco-alkylmethyl-amine | N,N-Dicoco-alkylmethyl-amine | N,N-Dicoco-alkylmethyl-amine | N,N-Dihydro-genated tallow alkylmethylamine |
| Distillation yield (%)[3] | 97.7 | 97.2 | 97.8 | 98.0 | 97.8 | — | — | — | — |
| Purity (%)[4] | 99.3 | 99.1 | 99.4 | 99.0 | 99.5 | 98.0 | 98.1 | 98.6 | 98.2 |
| Color (APHA)[5] | <10 | <10 | <10 | <10 | <10 | <30 | <30 | <30 | <30 |
| Smell[6] | o | o | o | o | o | o | o | o | o |

Note:

1) Weight percent of hydrogenation catalyst (including support) to the starting amine. Number in the parentheses means the concentration of the catalyst metal based on the amount of the starting amine (unit: ppm).

2) 37% aqueous solution (Examples 1, 3, 6, 7 and 9) or 50% aqueous solution (Examples 2, 4, 5 and 8). The amount of formaldehyde is in terms of mols per active hydrogen in the starting amine.

3) The dash indicates the absence of distillation.

4) Tertiary amine value/total amine value×100; The products of Examples 1 to 5 were purified by distillation, and those of Examples 6 to 9 were crude.

5) The products of Examples 1 to 5 were purified by distillation and those of Examples 6 to 9 were crude.

6) The samples were subjected to an organoleptic test consisting of 10 panelists, and those which were evaluated by at least 8 panelists as having no smell other than that of amines were given the mark "o".

TABLE 1-2

| | Example No. 10 | 11 | 12 | 13 | 14 | 15 | 16 |
|---|---|---|---|---|---|---|---|
| Starting amine | Octylamine | Octylamine | Octylamine | Octadecylamine | Dicocoalkylamine | Dihydrogenated tallowalkylamine | Dihydrogenated tallowalkylamine |
| Reaction condition | | | | | | | |
| Hydrogenation catalyst | | | | | | | |
| Type | 2% Pt-C | 5% Pd-C | 5% Pd-C | 10% Co-C | 2% Pt-C | 2% Pt-C | 5% Ru-C |
| Amount (%)[1] | 0.1 (20) | 0.1 (50) | 0.1 (50) | 5 (5,000) | 0.1 (20) | 0.1 (20) | 0.5 (250) |
| Aldehyde[2] | | | | | | | |
| Type | Propanal | Benzaldehyde | P-Tolualdehyde | Acetaldehyde | Acetaldehyde | Octadecanal | 2-Hexenal |
| Amount | 1.05 | 1.05 | 1.05 | 1.05 | 1.0 | 1.0 | 1.0 |
| Reaction temperature (°C) | 150 | 160 | 160 | 180 | 140 | 150 | 150 |
| Reaction pressure ($kg/cm^2 \cdot G$) | 30 | 20 | 20 | 50 | 15 | 30 | 30 |
| Reaction time (hr) | 4 | 5 | 5 | 5 | 4 | 4 | 4 |
| Analytical value of product | | | | | | | |
| Tertiary amine | N,N-Dipropyloctylamine | N,N-Dibenzyloctylamine | N,N-Di-p-methylbenzyloctylamine | N,N-Diethyloctadecylamine | N,N-Dicocoalkylethylamine | N,N-Dihydrogenated tallowalkyloctadecylamine | N,N-Dihydrogenated tallowalkylhexylamine |
| Distillation yield (%)[3] | 98.1 | 97.0 | 97.3 | 97.9 | — | — | — |
| Purity (%)[4] | 99.0 | 98.2 | 98.6 | 99.3 | 97.7 | 98.0 | 97.1 |
| Color (APHA)[5] | <10 | <10 | <10 | <10 | <30 | <30 | <30 |
| Smell[6] | o | o | o | o | o | o | o |

Notes:

1) Weight percent of hydrogenation catalyst (including support) to the starting amine. Number in the parentheses means the concentration of the catalyst metal based on the amount of the starting amine (unit: ppm).

2) Acetaldehyde was 60% aqueous solution (Examples 13 and 14). The amount of each aldehyde is in terms of mols per active hydrogen in the starting amine.

3) The dash indicates the absence of distillation.

4) Tertiary amine value/total amine value×100; The products of Examples 10 to 13 were purified by distillation, and those of Examples 14 to 17 were crude.

5) The products of Examples 10 to 13 were purified by distillation and those of Examples 14 to 17 were crude.

6) The samples were subjected to an organoleptic test consisting of 10 panelists, and those which were evaluated by at least 8 panelists as having no smell other than that of amines were given the mark "o".

Example 18 to 40

A 50 liter autoclave equipped with a stirrer, a hydrogen circulator with a condenser and a compressor was charged with 20 kg of a starting amine and a hydrogenation catalyst. The temperature in the autoclave was elevated to a predetermined level under agitation. After replacing the atmosphere in the autoclave with hydrogen, an additional volume of hydrogen was supplied to a predetermined level. The starting amine was hydrogenated while an aldehyde was pumped into the autoclave over a predetermined period. After the completion of the addition of the aldehyde, the reaction was continued for 30 minutes. The reaction was performed at a constant temperature and pressure while hydrogen was circulated and water that was condensed was removed from the reactor. After completion of the reaction, the hydrogenation catalyst was filtered off to obtain a crude tertiary amine. In some Examples, the crude amine was purified by vacuum distillation. The reaction conditions used in the respective Examples and the analytical values for the resulting products are shown in Table 2.

As is clear from Table 2, all the crude tertiary amines obtained were substantially colorless (APHA<30), and so were the purified tertiary amines. Neither the crude nor purified tertiary amines had a smell other than that of the amines. The product tertiary amines, whether they were crude or purified, had a purity of at least 98%.

The purified tertiary amines were stored at 50°C for 6 months. They were also subjected to a daylight exposure test outdoors for 6 months. In neither test did the amines undergo a change in appearance or smell.

TABLE 2-1

| | Example No. 18 | 19 | 20 | 21 | 22 | 23 | 24 |
|---|---|---|---|---|---|---|---|
| Starting amine | Dodecylamine | Dodecylamine | Octadecylamine | Cocoalkylamine | Tallowalkyl-amine | Tallowalkyl-amine | Rapeseed oil alkylamine |
| Reaction condition | | | | | | | |
| Hydrogenation catalyst | | | | | | | |
| Type | 5% Rh-C | 5% Pd-C | 5% Pd-C | 5% Pd-C | 5% Pd-C | 0.5% Pd-C | 5% Pd-C |
| Amount (%)[1] | 0.1 (50) | 0.1 (50) | 0.1 (50) | 0.1 (50) | 0.1 (50) | 0.3 (15) | 0.1 (50) |
| Aldehyde[2] | | | | | | | |
| Type | Formaldehyde | Formaldehyde | Formaldehyde | Formaldehyde | Formaldehyde | Formaldehyde | Formaldehyde |
| Amount | 1.05 | 1.05 | 1.0 | 1.05 | 1.0 | 1.0 | 1.0 |
| Rate of hydrogen circulation ($NM^3$/hr)[3] | 1 | 1 | 1 | 1 | 1 | 1 | 1 |
| Reaction temperature (°C) | 165 | 145 | 145 | 145 | 145 | 145 | 145 |
| Reaction pressure ($kg/m^2 \cdot G$) | 30 | 15 | 10 | 15 | 5 | 15 | 10 |
| Reaction time (hr) | 4 | 4 | 3 | 4 | 4 | 6 | 5 |
| Analytical value of product | | | | | | | |
| Tertiary amine | N,N-Dimethyl-dodecylamine | N,N-Dimethyl-dodecylamine | N,N-Dimethyl-octadecyl-amine | N,N-Dimethyl-cocoalkyl-amine | N,N-Dimethyl-tallowalkyl-amine | N,N-Dimethyl-tallowalkyl-amine | N,N-Dimethyl rapeseed oil alkylamine |
| Distillation yield (%) | 97.7 | 97.7 | 98.5 | 97.6 | 98.3 | 98.0 | 98.7 |
| Purity (%)[4] | 99.4 | 99.5 | 99.5 | 99.2 | 99.1 | 99.4 | 99.6 |
| Color (APHA)[5] | <10 | <10 | <10 | <10 | <10 | <10 | <10 |
| Smell[6] | o | o | o | o | o | o | o |

Note:

1) Weight percent of hydrogenation catalyst (including support) to the starting amine. Number in the parentheses means the concentration of the catalyst metal based on the amount of the starting amine (unit: ppm).

2) 47% aqueous solution (Examples 18, 21, 22 and 23) or 37% aqueous solution (Examples 19, 20 and 24). The amount of formaldehyde is in terms of mols per active hydrogen in the starting amine.

3) Based on 1 kg of the starting amine.

4) Tertiary amine value/total amine value×100.

5) The products of Examples 18 to 24 were purified by distillation.

6) The samples were subjected to an organoleptic test consisting of 10 panelists, and those which were evaluated by at least 8 panelists as having no smell other than that of amines were given the mark "o".

TABLE 2-2

| | Example No. 25 | 26 | 27 | 28 | 29 | 30 | 31 |
|---|---|---|---|---|---|---|---|
| Starting amine | Dicocoalkyl-amine | Dicocoalkyl-amine | Dihydrogenated tallowalkylamine | Dihydrogenated tallowalkyl-amine | N-Tallowalkyl-ethylene-diamine | N-Cocoalkyl-propylene-diamine | N-Tallowalkyl-dipropylene-triamine |
| Reaction condition | | | | | | | |
| Hydrogenation catalyst | | | | | | | |
| Type | 10% Ni-C | 10% Co-C | 10% Ni-C | 5% Pd-C | 5% Pd-C | 5% Pd-C | 5% Pd-C |
| Amount (%)[1] | 2 (2,000) | 3 (3,000) | 2 (2,000) | 0.1 (50) | 0.2 (100) | 0.2 (100) | 0.2 (100) |
| Aldehyde[2] | | | | | | | |
| Type | Formaldehyde | Formaldehyde | Formaldehyde | Formaldehyde | Formaldehyde | Formaldehyde | Formaldehyde |
| Amount | 1.2 | 1.2 | 1.5 | 1.0 | 1.0 | 1.0 | 1.0 |
| Rate of hydrogen circulation ($NM^3/hr$)[3] | 1 | 1 | 1 | 1 | 1 | 1 | 1 |
| Reaction temperature (°C) | 180 | 180 | 180 | 170 | 150 | 150 | 150 |
| Reaction pressure ($kg/cm^2 \cdot G$) | 10 | 15 | 15 | 10 | 15 | 15 | 15 |
| Reaction time (hr) | 5 | 6 | 4 | 4 | 5 | 5 | 5 |
| Analytical value of product | | | | | | | |
| Tertiary amine | N,N-Dicoco-alkylmethyl-amine | N,N-Dicoco-alkylmethyl-amine | N,N-Dihydro-genated tallow-alkylmethyl-amine | N,N-Dihydro-genated tallow-alkylmethyl-amine | N-Hydrogenated tallowalkyl N,N',N'-tri-methylethylene-diamine | N-Cocoalkyl N,N',N'-tri-methylpropylene-diamine | N-Hydrogenated tallowalkyl-N,N',N',N'',N''-pentamethyldi-propylene-triamine |
| Distillation yield (%)[4] | — | — | — | — | 97.0 | 98.1 | 96.1 |
| Purity (%)[5] | 98.2 | 98.0 | 98.1 | 98.6 | 98.0 | 98.4 | 98.1 |
| Color (APHA)[6] | <30 | <30 | <30 | <30 | <30 | <30 | <30 |
| Smell[7] | o | o | o | o | o | o | o |

Note: 1) Weight percent of hydrogenation catalyst (including support) to the starting amine. Number in the parentheses means the concentration of the catalyst metal based on the amount of the starting amine (unit: ppm).

2) 37% aqueous solution (Examples 25, 26, 27 and 28) or 47% aqueous solution (Examples 29, 30 and 31). The amount of formaldehyde is in terms of mols per active hydrogen in the starting amine.

3) Based on 1 kg of the starting amine.

4) The dash indicates the absence of distillation.

5) Tertiary amine value/total amine value×100.

6) The products of Examples 25 to 28 were crude and those of Examples 29 to 31 were purified by distillation.

7) The samples were subjected to an organoleptic test consisting of 10 panelists, and those which were evaluated by at least 8 panelists as having no smell other than that of amines were given the mark "o".

TABLE 2-3

| | Example No. | | | | | | |
|---|---|---|---|---|---|---|---|
| | 32 | 33 | 34 | 35 | 36 | 38 | 39 |
| Starting amine | Dodecylamine | Tetradecyl-amine | Octadecyl-amine | 9-Octadecenyl-amine | Dicocoalkyl-amine | Dihydrogenated tallowalkylamine | Dihydrogenated tallowalkylamine |
| Reaction condition | | | | | | | |
| Hydrogenation catalyst | | | | | | | |
| Type | 5% Ru-C | 10% Co-C | 5% Rh-C | 2% Pt-C | 10% Ni-C | 5% Pd-C | 5% Pd-C |
| Amount (%)[1] | 0.5 (250) | 5 (5,000) | 0.1 (50) | 0.1 (20) | 3 (3,000) | 0.1 (50) | 0.1 (50) |
| Aldehyde[2] | | | | | | | |
| Type | Acetaldehyde | Acetaldehyde | Propanal | Propanal | p-Methoxybenz-aldehyde | p-Chlorobenz-aldehyde | 1-Naphthaldehyde |
| Amount | 1.05 | 1.05 | 1.05 | 1.05 | 1.0 | 1.0 | 1.0 |
| Rate of hydrogen circulation ($NM^3$/hr)[3] | 1 | 1 | 1 | 1 | 1 | 1 | 1 |
| Reaction temperature (°C) | 160 | 160 | 150 | 150 | 160 | 150 | 150 |
| Reaction pressure ($kg/cm^2 \cdot G$) | 30 | 50 | 30 | 15 | 30 | 15 | 15 |
| Reaction time | 5 | 5 | 5 | 4 | 5 | 4 | 4 |
| Analytical value of product | | | | | | | |
| Tertiary amine | N,N-Diethyl-dodecylamine | N,N-Diethyl-tetradecyl-amine | N,N-Dipropyl-octadecyl-amine | N,N-Dipropyl-octadecyl-amine | N,N-Dicocoalkyl p-methoxy-benzylamine | N,N-Dihydro-genated tallow-alkyl p-chloro-benzylamine | N,N-Dihydro-genated tallow-alkyl 1-naphthyl-methylamine |
| Distillation yield (%)[4] | 97.0 | 97.4 | 97.2 | 98.0 | — | — | — |
| Purity (%)[5] | 98.2 | 98.0 | 99.1 | 99.1 | 98.4 | 98.9 | 98.3 |
| Color (APHA)[6] | <10 | <10 | <10 | <10 | <30 | <30 | <30 |
| Smell[7] | o | o | o | o | o | o | o |

Notes:

1) Weight percent of hydrogenation catalyst (including support) to the starting amine. Number in the parentheses means the concentration of the catalyst metal based on the amount of the starting amine (unit: ppm).

2) Acetaldehyde was 60% aqueous solution (Examples 32 and 33). The amount of each aldehyde is in terms of mols per active hydrogen in the starting amine.

3) Based on 1 kg of the starting amine.

4) The dash indicates the absence of distillation.

5) Tertiary amine value/total amine value×100.

6) The products of Examples 32 to 35 were purified by distillation and those of Examples 36 to 40 were crude.

7) The samples were subjected to an organoleptic test consisting of 10 panelists, and those which were evaluated by at least 8 panelists as having no smell other than that of amines were given the mark "o".

Example 41

A 2 liter conical flask was charged with 500 g of dodecylamine, which was heated to 70°C under agitation. The flask was also charged with 219 g of 37% aqueous formaldehyde (0.5 mol per mol of the active hydrogen in dodecylamine) that was added dropwise over a period of 30 minutes. After the addition of aqueous formaldehyde, the mixture was stirred for 1 hour at 90°C, and thereafter, the aqueous layer was separated.

An autoclave which was the same as used in Example 1 was charged with the condensation product of dodecylamine with formaldehyde, and a hydrogenation catalyst [5% Pd-C: 0.5 g, or 0.1 wt%. for the starting amine (i.e., 50 ppm of Pd based on the amount of starting amine)]. The mixture was heated at 160°C under agitation with the turbine stirrer at 900 rpm. After replacing the system atmosphere with hydrogen, an additional volume of hydrogen was supplied until the gauge pressure in the reactor reached 10 $kg/cm^2$. Hydrogen absorption began immediately after the supply of hydrogen, and its pressure was held constant throughout the reaction. Two hours later, the hydrogen absorption ceased. Thereafter, 219 g of 37% aqueous formaldehyde (0.5 mol per mol of the active hydrogen in dodecylamine) was pumped in over a period of 2 hours, while the hydrogen pressure was kept at 10 $kg/cm^2G$ (gauge). Under the same conditions, the reaction was continued for 1 hour. After the completion of the reaction, the hydrogenation catalyst was filtered off and the aqueous layer was separated to obtain a crude tertiary amine. This amine was substantially colorless (APHA<30). It did not have a smell other than that of amines. The purity of the crude product was 97.4%. The crude amine was purified by vacuum distillation. The distillation yield was 95.2%. The purified amine had a purity of 98.1% and was completely colorless (APHA<10).

The purified tertiary amine was stored at 50°C for 6 months. It was also subjected to a daylight exposure test outdoors for 6 months. In either test, the amine experienced no change in appearance or smell.

This experiment shows that a tertiary amine of good quality can be obtained in high yield even if part of the formaldehyde is first added and the remainder is continuously added in the subsequent hydrogenation reaction.

Example 42

A hydrogenation reaction was conducted as in Example 41. Dodecylamine (500 g) was used as the starting amine. As an aldehyde, 60% aqueous acetaldehyde was added dropwise in an amount of 198 g (0.5 mol per mol of the active hydrogen in dodecylamine). In the subsequent reaction, the same amount of 60% aqueous acetaldehyde was pumped into the reaction system.

The resulting crude tertiary amine was substantially colorless (APHA<30). It had no smell other than that of amines, and its purity was 97.7%. The crude product was purified by vacuum distillation. The distillation yield was 96.1%. The purified tertiary amine had a purity of 98.4% and was completely colorless (APHA<10) and transparent.

The purified tertiary amine was stored at 50°C for 6 months. It was also subjected to a daylight exposure test outdoors for 6 months. In neither test did the amine undergo any change in appearance or smell.

This experiment also shows that a tertiary amine of good quality can be obtained in high yield even if part of an aldehyde is first added and the remainder is continuously added in the subsequent hydrogenation reaction.

Comparative Examples 1 to 16

In Comparative Examples 1 to 9, the hydrogenation reaction was conducted as in Example 1, except that the amount of the starting amine used was 800 g (Comparative Examples 1 to 5) or 400 g (Comparative Examples 6 to 9), and the catalyst used was Raney nickel (Comparative Examples 1 and 6), palladium black (Comparative Examples 2 and 7), platinum supported on silica (Comparative Examples 3 and 8), palladium supported on diatomaceous earth (Comparative Examples 4 and 9), or platinum oxide (Comparative Example 5).

In Comparative Examples 10 to 14, the hydrogenation reaction was conducted as in Example 18, except that the catalyst used was platinum oxide (Comparative Example 13) and Raney nickel (Comparative Examples 10, 11, 12 and 14).

In Comparative Examples 15 and 16, the reaction was started with all the necessary amount of an aldehyde. The catalyst used was palladium on carbon which is included within the scope defined by the present invention. The reductive alkylation reaction was conducted as in Example 1, but no aldehyde was added during the reaction.

In each Comparative Example, the resulting crude tertiary amine was treated as in Examples 1 to 42: the hydrogenation catalyst was filtered off, and after separating the aqueous layer, the product was purified by vacuum distillation. The reaction conditions used in the respective Comparative Examples and the analytical values for the resulting products are shown in Table 3.

TABLE 3-1

| | Comparative Example No. | | | | | | | | |
|---|---|---|---|---|---|---|---|---|---|
| | 1 | 2 | 3 | 4 | 5 | 6 | 7 | 8 | 9 |
| Starting amine | Dodecylamine | Dodecylamine | Dodecylamine | Dodecylamine | Dodecylamine | Dodecylamine | Dodecylamine | Octadecylamine | Dihydrogenated tallowalkylamine |
| Reaction condition | | | | | | | | | |
| Hydrogenation catalyst | | | | | | | | | |
| Type | Raney-Ni | Pd Black | 2% $Pt-SiO_2$ | 5% Pd-Diatomaceous earth | $PtO_2$ | Raney-Ni | Pd Black | 2% $Pt-SiO_2$ | 5% Pd-Diatomaceous earth |
| Amount (%)[1] | 0.8 | 0.6 | 0.3 | 0.6 | 0.1 | 0.8 | 0.6 | 0.2 | 0.5 |
| Aldehyde[2] | | | | | | | | | |
| Type | Formaldehyde | Formaldehyde | Formaldehyde | Formaldehyde | Formaldehyde | Acetaldehyde | Propanal | Octadecanal | Acetaldehyde |
| Amount | 1.05 | 1.05 | 1.05 | 1.05 | 1.05 | 1.05 | 1.05 | 1.05 | 1.0 |
| Rate of hydrogen circulation ($NM^3$/hr)[3] | 0 | 0 | 0 | 0 | 0 | 0 | 0 | 0 | 0 |
| Reaction temperature (°C) | 150 | 150 | 150 | 150 | 150 | 150 | 150 | 150 | 150 |
| Reaction pressure ($kg/cm^2 \cdot G$) | 30 | 15 | 15 | 15 | 15 | 30 | 15 | 15 | 15 |
| Reaction time (hr) | 5 | 5 | 5 | 5 | 5 | 5 | 5 | 5 | 5 |
| Analytical value of product | | | | | | | | | |
| Tertiary amine | N,N-Dimethyl-dodecylamine | N,N-Dimethyl-dodecylaine | N,N-Dimethyl-dodecylamine | N,N-Dimethyl-dodecylamine | N,N-Dimethyl-dodecylamine | N,N-Diethyl-dodecylamine | N,N-Dipropyl-dodecylamine | Trioctadecyl-amine | N,N-Dihydro-genated tallow-alkylethyl-amine |
| Distillation yield (%)[4] | 74.3 | 91.0 | 91.2 | 86.0 | 89.4 | 76.0 | 92.2 | 93.0 | — |
| Purity (%)[5] | 96.5 | 98.1 | 98.6 | 96.6 | 97.0 | 96.7 | 98.6 | 99.0 | 96.6 |
| Color (APHA) | | | | | | | | | |
| Just after distillation | 30 | 10 | 10 | 10 | 10 | 30 | 10 | 10 | — |
| 1 month later | 90 | 80 | 70 | 100 | 120 | 80 | 60 | 60 | 120 |
| Smell[6] | x | x | x | x | x | x | x | x | x |

Notes:
1) Weight percent to the starting amine
2) Formaldehyde was 37% aqueous solution and acetaldehyde was 60% aqueous solution. The amount of each aldehyde is in terms of mols per active hydrogen in the starting amine.
3) Based on 1 kg of the starting amine.
4) The product of Comparative Example 9 was not distilled.
5) Tertiary amine value/total amine value×100.
6) The samples were subjected to an organoleptic test consisting of 10 panelists, and those which were evaluated by at least 8 panelists as having a smell other than that of amines were given the mark "x".

TABLE 3-2

| | Comparative Example No. | | | | | | |
|---|---|---|---|---|---|---|---|
| | 10 | 11 | 12 | 13 | 14 | 15 | 16 |
| Starting amine | Dodecylamine | Octadecylamine | Dodecylamine | Octadecylamine | Dihydrogenated tallowalkylamine | Dodecylamine | Dodecylamine |
| Reaction condition | | | | | | | |
| Hydrogenation catalyst | | | | | | | |
| Type | Raney-Ni | Raney-Ni | Raney-Ni | $PtO_2$ | Raney-Ni | 10% Pd-C | 5% Pd-C |
| Amount (%)[1] | 0.8 | 0.8 | 0.8 | 0.1 | 0.8 | 2.0 | 1.0 |
| Aldehyde[2] | | | | | | | |
| Type | Formaldehyde | Formaldehyde | Acetaldehyde | Benzaldehyde | Acetaldehyde | Formaldehyde | Acetaldehyde |
| Amount | 1.05 | 1.05 | 1.05 | 1.05 | 1.0 | 1.05 | 1.05 |
| Rate of hydrogen circulation ($NM^3/hr$)[3] | 1 | 1 | 1 | 1 | 1 | 0 | 0 |
| Reaction temperature (°C) | 150 | 150 | 160 | 150 | 160 | 150 | 150 |
| Reaction pressure ($kg/cm^2 \cdot G$) | 15 | 15 | 30 | 15 | 30 | 30 | 30 |
| Reaction time (hr) | 5 | 5 | 4 | 4 | 4 | 5 | 5 |
| Analytical value of product | | | | | | | |
| Tertiary amine | N,N-Dimethyl-dodecylamine | N,N-Dimethyl-octadecylamine | N,N-Diethyl-dodecylamine | N,N-Dicyclo-hexylmethylocta-decylamine | N,N-Dihydro-genated tallow-alkylethylamine | N,N-Dimethyl-dodecylamine | N,N-Diethyl-dodecylamine |
| Distillation yield (%)[4] | 91.0 | 90.2 | 90.3 | 89.7 | — | 72.0 | 69.9 |
| Purity (%)[5] | 97.7 | 97.4 | 95.8 | 94.4 | 97.0 | 83.4 | 86.1 |
| Color (APHA) | | | | | | | |
| Just after distillation | 10 | 10 | 10 | 20 | — | 180 | 120 |
| 1 month later | 90 | 70 | 90 | 100 | 60 | 250 | 300 |
| Smell[6] | x | x | x | x | x | x | x |

Notes:
[1] Weight percent to the starting amine.
[2] Formaldehyde was 37% aqueous solution and acetaldehyde was 60% aqueous solution. The amount of each aldehyde is in terms of mols per active hydrogen in the starting amine.
[3] Based on 1 kg of the starting amine.
[4] The product of Comparative Example 14 was not distilled.
[5] Tertiary amine value/total amine value×100.
[6] The samples were subjected to an organoleptic test consisting of 10 panellists, and those which were evaluated by at least 8 panelists as having a smell other than that of amines were given the mark "x".

As is apparent from Table 3, the distillation yields and the purities of the product tertiary amines were lower than those obtained in Examples 1 to 42. The purified tertiary amines were colorless and transparent just after distillation, but upon standing at room temperature for 1 month, they acquired appreciable shades of color. This color staining proceeded further when the amines were left at 50°C or exposed to a daylight exposure test. The amines produced in the Comparative Examples smelled of aldehydes or had other unpleasant odors just after distillation. The undesired odors became intense when they were left at 50°C or subjected to a daylight exposure test.

The results of Comparative Examples 1 to 9 show that catalysts outside the scope of the present invention do not give good results in reductive alkylation of amines. Data of Comparative Examples 10 to 14 show that the effectiveness of catalysts outside the scope of the present invention is not enhanced to the desired level even if hydrogen is circulated for removing water during the reaction. Comparative Examples 15 and 16 show that the aldehyde must be continuously added throughout the reductive alkylation reaction.

**Claims**

1. A process for producing a tertiary amine by alkylating an amine of formula (I)

$$R^1R^2N[(CH_2)_nNH]_mR^3 \qquad (I)$$

wherein $R^1$ represents a linear or branched alkyl or alkenyl group having from 8 to 24 carbon atoms; $R^2$ and $R^3$ each represents a hydrogen atom, or a linear or branched alkyl or alkenyl group having from 8 to 24 carbon atoms; m is 0 or an integer of 1 to 5 and n is 2 or 3, provided that when m=0, at least one of $R^2$ and $R^3$ represents a hydrogen atom with an aldehyde of formula (II)

$$R^4\overset{\overset{\displaystyle O}{\|}}{C}H \qquad (II)$$

wherein $R^4$ represents a hydrogen atom, a saturated or unsaturated, linear, branched or cyclic aliphatic hydrocarbon group having from 1 to 24 total carbon atoms, an aromatic group-substituted aliphatic hydrocarbon group having 24 or less total carbon atoms, or a substituted or unsubstituted aromatic hydrocarbon group having 24 or less total carbon atoms, wherein a hydrogenation catalyst having from 0.1 to 10 wt% of at least one of Co, Ni, Ru, Rh, Pd, and Pt supported on pulverized or granular carbon is provided in a reaction zone at the concentration of from 5 to 5,000 ppm of the catalyst metal based on the amount of the amine of formula (I), and the reaction is performed at a temperature of from 80 to 250°C and a hydrogen pressure of at least 2 kg/cm$^2$ (gauge) while the aldehyde is continuously supplied to the reaction zone.

2. A process as in claim 1, wherein the hydrogen pressure is from 5 to 50 kg/cm$^2$ (gauge).

3. A process as in claim 1, wherein the reaction is performed at a temperature of from 100 to 250°C.

4. A process as in claim 2, wherein the reaction is performed at a temperature of from 100 to 250°C.

5. A process as in claim 1, wherein the aldehyde is used in an amount of from 1 to 1.5 mols per mol of active hydrogen of the amine of the formula (I).

6. A process as in claim 5, wherein the aldehyde is used in an amount of from 1 to 1.05 mols per mol of active hydrogen of the amine of the formula (I).

**Patentansprüche**

1. Verfahren zur Herstellung eines tertiären Amins durch
— Alkylieren eines Amins der Formel (I)

$$R^1R^2N[(CH_2)_nNH]_mR^3 \qquad (I)$$

wobei $R^1$ eine geradkettige oder verzweigte Alkyl- oder Alkenylgruppe mit 8 bis 24 Kohlenstoffatomen darstellt; $R^2$ und $R^3$ stellen jeweils ein Wasserstoffatom, oder eine geradkettige oder verzweigte Alkyl- oder Alkenylgruppe mit 8 bis 24 Kohlenstoffatomen dar; m ist 0 oder eine ganze Zahl zwischen 1 und 5 und n ist 2 oder 3, vorausgesetzt, dass, wenn m=0, zumindest eine der beiden Gruppen $R^2$ und $R^3$ ein Wasserstoffatom darstellt,
— mit einer Aldehydverbindung der Formel (II)

$$R^4\overset{\overset{\displaystyle O}{\|}}{C}H \qquad (II)$$

wobei $R^4$ ein Wasserstoffatom, eine gesättigte oder ungesättigte, geradkettige, verzweigte oder zyklische,

aliphatische Kohlenwasserstoffgruppe mit insgesamt 1 bis 24 Kohlenstoffatomen, eine mit einer aromatischen Gruppe substituierte aliphatische Kohlenwasserstoffgruppe mit insgesamt 24 oder weniger Kohlenstoffatomen, oder eine substituierte oder unsubstituierte aromatische Kohlenwasserstoffgruppe mit insgesamt 24 oder weniger Kohlenstoffatomen darstellt,

— wobei ein Hydrierkatalysator, der zwischen 0,1 und 10 Gew.% zumindest eines Elementes aus der Reihe Co, Ni, Ru, Rh, Pd und Pt, getragen von pulverisiertem oder gekörntem Kohlenstoff, enthält, einer Reaktionszone zugeführt wird, wobei die Konzentration des katalystisch aktiven Metalls zwischen 5 und 5.000 ppm, bezogen auf die Menge des Amins mit der Formel (I), beträgt, und die Reaktion bei einer Temperatur von 80 bis 250°C und einem Wasserstoffdruck von mindestens 2 kg/cm$^2$ (Gauge) durchgeführt wird, während die Aldehydverbindung kontinuierlich der Reaktionszone zugeführt wird.

2. Verfahren nach Anspruch 1, wobei der Wasserstoffdruck im Bereich zwischen 5 und 50 kg/cm$^2$ (Gauge) liegt.

3. Verfahren nach Anspruch 1, wobei die Reaktion bei einer Temperatur zwischen 100 und 250°C durchgeführt wird.

4. Verfahren nach Anspruch 2, wobei die Reaktion bei einer Temperature zwischen 100 und 250°C durchgeführt wird.

5. Verfahren nach Anspruch 1, wobei die Aldehydverbindung in einer Menge von 1 bis 1,5 Mol pro Mol aktiven Wasserstoffes des Amins der Formel (I) eingesetzt wird.

6. Verfahren nach Anspruch 5, wobei die Aldehydverbindung in einer Menge von 1 bis 1,05 Mol pro Mol aktiven Wasserstoffes des Amins der Formel (I) eingesetzt wird.

**Revendications**

1. Un procédé de production d'une amine tertiaire par alkylation d'une amine de formule (I)

$$R^1R^2N[(CH_2)_nNH]_mR^3 \qquad (I)$$

dans laquelle R$^1$ représente un groupe alkyle ou alcényle linéaire ou ramifié ayant de 8 à 24 atomes de carbone; R$^2$ et R$^3$ représentent chacun un atome d'hydrogène ou un groupe alkyle ou alcényle linéaire ou ramifié ayant de 8 à 24 atomes de carbone; m est 0 ou un nombre entier de 1 à 5 et n est 2 ou 3, à la condition que lorsque m=0, l'une au moins des substituants R$^2$ et R$^3$ représente un atome d'hydrogène, avec un aldéhyde de formule (II)

$$R^4CH{=}O \qquad (II)$$

dans laquelle R$^4$ représente un atome d'hydrogène, un groupe hydrocarboné aliphatique saturé ou insaturé, linéaire, ramifié ou cyclique ayant de 1 à 24 atomes de carbone au total, un groupe hydrocarboné aliphatique substitué par un groupe aromatique ayant 24 atomes de carbone au total ou moins ou un groupe hydrocarboné aromatique substitué ou non substitué ayant 24 atomes de carbone au total ou moins, procédé selon lequel un catalyseur d'hydrogénation ayant de 0,1 à 10% en poids de l'un au moins des Co, Ni, Ru, Rh, Pd et Pt sur support de charbon pulvérisé ou granulaire est fourni dans une zone de réaction à la concentration de 5 à 5 000 ppm de métal catalytique basée sur la quantité de l'amine de formule (I), et la réaction est conduite à une température de 80 à 250°C et à une pression d'hydrogène d'au moins 2 kg/cm$^2$ (au manomètre) pendant que l'aldehyde est fourni en continu dans la zone de réaction.

2. Un Procédé selon la revendication 1, selon lequel la pression d'hydrogène est de 5 à 50 kg/cm$^2$ (au manomètre).

3. Un procédé selon la revendication 1, selon lequel la réaction est conduite à une température de 100 à 250°C.

4. Un procédé selon la revendication 2, selon lequel la réaction est conduite à une température de 100 à 250°C.

5. Un procédé selon la revendication 1, selon lequel l'aldéhyde est utilisé en quantité de 1 à 1,5 mole par mole d'hydrogène actif de l'amine de formule (I).

6. Un procédé selon la revendication 5, selon lequel l'aldéhyde est utilisé en quantité de 1 à 1,05 mole par mole d'hydrogène actif de l'amine de formule (I).